# EUROPÄISCHE PATENTSCHRIFT

(11) **EP 0 984 948 B1**
(45) Veröffentlichungstag und Bekanntmachung des Hinweises auf die Patenterteilung: **24.09.2003**
(21) Anmeldenummer: 98922650.1
(22) Anmeldetag: 06.04.1998
(51) Int. Cl.: C07D 271/07, A01N 43/82

(54) **SULFONYLOXADIAZOLONE**
SULFONYLOXADIAZOLONES
SULFONYLOXADIAZOLONES

(30) Priorität: 18.04.1997 DE 19716260
(43) Veröffentlichungstag der Anmeldung: 15.03.2000
(73) Patentinhaber: Bayer CropScience AG, 40789 Monheim (DE)
(72) Erfinder: ASSMANN, Lutz, D-23701 Eutin (DE); GERDES, Peter, D-52080 Aachen (DE); STENZEL, Klaus, D-40595 Düsseldorf (DE); DENG, Chuan-Zheng, Shanghai 200335 (CN); YUAN, Li-Ping, Shanghai 200233 (CN); ZHAO, Jian-Mei, Shanghai 200237 (CN)
(86) Internationale Anmeldenummer: EP9801990
(87) Internationale Veröffentlichungsnummer: WO98047883

(56) Entgegenhaltungen:
- DE-A- 2 405 324
- US-A- 3 958 000
- US-A- 5 292 762

## Beschreibung

Die vorliegende Erfindung betrifft neue Sulfonyloxadiazolone, ein Verfahren zu deren Herstellung und deren Verwendung als Fungizide im Pflanzenschutz.

Es sind bereits bestimmte Sulfonyloxadiazolone, wie z.B. das 4-[(4-Chlorphenyl)-sulfonyl]-3-(2,4,6-trimethylphenyl)-1,2,4-oxadiazol-5(4H)-on, bekannt (vgl. Zh. Org. Khim 27 (1991), 1262-1270). Eine biologische Wirkung dieser Verbindungen ist aber bisher noch nicht beschrieben worden.

Weiterhin betreffen die DE - A 2 405 324 und die US - A 3 958 000 1,2,4-Oxadiazolone mit fungiziden Eigenschaften. Es werden aber keine Verbindungen dieses Typs beschrieben, in denen der Rest in der 4-Position für eine Sulfonyl - gruppe steht.

Schließlich sind aus der US - A 5 292 762 bestimmte 1,3,4 - Oxadiazolone bekannt. Entsprechende 1,2,4 - Heterocyclen werden dort jedoch nicht offenbart.

Es wurden nun neue Sulfonyloxadiazolone der Formel 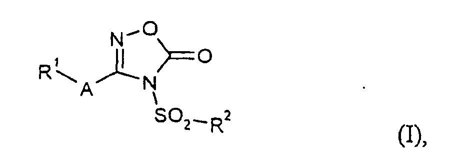 in welcher
- A: für Sauerstoff, Schwefel, -SO-, -SO₂- oder 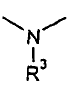 steht, wobei
R³ für Wasserstoff oder Alkyl mit I bis 4 Kohlenstoffatomen steht,
- R¹: für jeweils gegebenenfalls einfach bis dreifach, gleichartig oder verschieden durch Halogen oder Alkyl mit 1 bis 3 Kohlenstoffatomen. substituiertes Cycloalkyl mit 3 bis 8 Kohlenstoffatomen oder Cycloalkenyl mit 3 bis 8 Kohlenstoffatomen steht oder
- R¹: für Aryl mit 6 bis 10 Kohlenstoffatomen steht, wobei jeder dieser Reste einfach bis dreifach, gleichartig oder verschieden substituiert sein kann durch
Halogen, Nitro, Carbamoyl;
jeweils geradkettiges oder verzweigtes Alkyl, Alkoxy, Alkylthio oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio oder Halogenalkylsulfonyl mit jeweils 1 bis 6 Kohlenstoffatomen und 1 bis 5 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 5 gleichen oder verschiedenen Halogenatomen;
jeweils geradkettiges oder verzweigtes Alkylamino, Dialkylamino, Alkylcarbonyl, Alkoxycarbonyl, Hydroximinoalkyl oder Alkoximinoalkyl mit jeweils 1 bis 6 Kohlenstoffatomen in den einzelnen Alkylteilen;
jeweils gegebenenfalls einfach bis vierfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 5 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
und/oder
Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio, wobei diese aromatischen oder heterocyclischen Reste einfach bis dreifach, gleichartig oder verschieden substituiert sein können durch
Halogen, Nitro, geradkettiges oder verzweigtes Alkyl mit 1 bis 4 Kohlenstoffatomen,
geradkettiges oder verzweigtes Halogenalkyl mit 1 bis 4 Kohlenstoffatomen und 1 bis 5 gleichen oder verschiedenen Halogenatomen,
geradkettiges oder verzweigtes Alkoxy oder Alkylthio mit 1 bis 4 Kohlenstoffatomen,
und/oder durch geradkettiges oder verzweigtes Halogenalkoxy mit 1 bis 4 Kohlenstoffatomen und 1 bis 9 gleichen oder verschiedenen Halogenatomen,
und
- R²: für Alkyl mit 1 bis 4 Kohlenstofxatomen, Alkenyl mit 2 bis 4 Kohlen stoffätomen, Halogenalkyl mit jeweils 1 bis 4 Kohlenstoffatomen und 1 bis 5 gleichen oder verschiedenen Halogenatomen, Halogenalkenyl mit jeweils 2 bis 4 Kohleastoffatomen und 1 bis 5 gleichen oder verschiedenen Halogenatomen oder für Dialkylamino mit jeweils 1 bis 4 Kohlenstoffatomen in den beiden Alkylgruppen steht, oder
- R²: für Aryl mit 6 bis 10 Kohlenstoffatomen steht, wobei diese Reste einfach bis dreifach, gleichartig oder verschieden substituiert sein können durch Nitro, Halogen, Alkyl mit 1 bis 4 Kohlenstoffatomen, Alkoxy mit 1 bis 4 Kohlenstoffätomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen und/oder Halogenalkylthio mit 1 bis 4 Kohlenstoffatomen und 1 bis 5 gleichen oder verschiedenen Halogenatomen
gefunden.

Weiterhin wurde gefunden, daß man Sulfonyloxadiazolone der Formel (I) erhält, wenn man Oxadiazolone der Formel 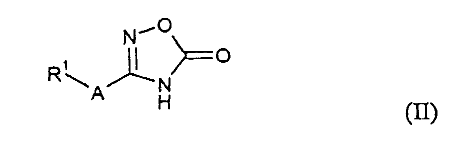 in welcher
- A und R¹: die oben angegebenen Bedeutungen haben,
mit Sulfonsäurehalogeniden der Formel

R²-SO₂-X (III)

in welcher
- R²: die oben angegebene Bedeutung hat und
- X: für Halogen steht,
gegebenenfalls in Gegenwart eines Säurebindemittels und gegebenenfalls in Gegenwart eines Verdünnungsmittels sowie gegebenenfalls in Gegenwart eines Katalysators umsetzt.

Schließlich wurde gefunden, daß die Sulfonyloxadiazolone der Formel (I) sehr gute fungizide Eigenschaften besitzen und im Pflanzenschutz eingesetzt werden können.

Überraschenderweise zeigen die erfindungsgemäßen Sulfonyloxadiazolone der Formel (I) eine bessere Wirksamkeit gegen unerwünschte Mikroorganismen, insbesondere Pilze, als die konstitutionell ähnlichsten, vorbekannten Stoffe gleicher Wirkungsrichtung.

Die erfindungsgemäßen Stoffe sind durch die Formel (I) allgemein definiert.

Heterocyclyl steht für ringförmige Verbindungen, in denen mindestens ein Ringglied ein Heteroatom, d. h. ein von Kohlenstoff verschiedenes Atom, ist. Enthält der Ring mehrere Heteroatome, so können diese gleich oder verschieden sein. Heteroatome sind vorzugsweise Sauerstoff, Stickstoff oder Schwefel. Enthält der Ring mehrere Sauerstoffatome, so stehen diese nicht benachbart.
- A: steht auch bevorzugt für Sauerstoff, Schwefel, -SO-, -SO₂- oder 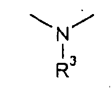
- R³: steht bevorzugt für Wasserstoff oder Methyl.
- R¹: steht bevorzugt für jeweils gegebenenfalls einfach bis dreifach durch Chlor, Methyl, Ethyl, n- oder i-Propyl substituiertes Cyclopropyl, Cyclobutyl, Cyclopentyl, Cyclohexyl, Cyclopentenyl oder Cyclohexenyl.
- R¹: steht auch bevorzugt für Phenyl, das einfach bis dreifach, gleichartig oder verschieden substituiert sein kann durch
Fluor, Chlor, Brom, Nitro, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl, Methoximinomethyl, Ethoximinomethyl, Methoximinoethyl oder Ethoximinoethyl,
gegebenenfalls einfach bis vierfach, gleich oder verschieden durch Fluor, Chlor, Methyl oder Trifluormethyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl,
und/oder
durch Phenyl, Phenoxy, Phenylthio, Benzyl, Benzyloxy, Benzylthio, Pyridyl, Pyrimidinyl oder Thienyl, wobei diese Reste einfach bis dreifach, gleichartig oder verschieden substituiert sein können durch
Fluor, Chlor, Brom, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Trifluormethyl, Trifluorethyl, Difluormethoxy, Trifluormethoxy, Difluorchlormethoxy und/oder Trifluorethoxy.
- R²: steht bevorzugt für Methyl, Ethyl, n- oder i-Propyl, n-, i-, s- oder t-Butyl, Dimethylamino, Diethylamino oder Methyl-ethylamino,
- R²: steht auch bevorzugt für Phenyl, das einfach bis dreifach, gleichartig oder verschieden substituiert sein kann durch Fluor, Chlor, Brom, Nitro, Methyl, Ethyl, Methoxy, Ethoxy, Methylthio, Ethylthio, Trifluormethyl, Trifluorethyl, Difluormethoxy, Trifluormethoxy, Difluorchlormethoxy, Trifluorethoxy, Difluormethylthio, Difluorchlormethylthio und/oder Trifluormethylthio.
- A: steht besonders bevorzugt für Sauerstoff.
- R¹: steht besonders bevorzugt für jeweils gegebenenfalls einfach bis dreifach durch Chlor, Methyl, Ethyl, n- oder i-Propyl substituiertes Cyclopropyl, Cyclobutyl, Cyclopentyl, Cyclohexyl, Cyclopentenyl oder Cyclohexenyl.
- R¹: steht auch besonders bevorzugt für Phenyl, das einfach bis dreifach, gleichartig oder verschieden substituiert sein kann durch
Fluor, Chlor, Brom, Nitro, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio oder Trifluormethylsulfonyl, Methylamino, Ethylamino, n- oder i-Propylamino, Dimethylamino, Diethylamino, Acetyl, Propionyl, Methoxycarbonyl, Ethoxycarbonyl, Hydroximinomethyl, Hydroximinoethyl. Methoximinomethyl, Ethoximinomethyl, Methoximinoethyl oder Ethoximinoethyl,
gegebenenfalls einfach bis vierfach, gleich oder verschieden durch Fluor, Chlor, Methyl oder Trifluormethyl substituiertes, jeweils zweifach verknüpftes Trimethylen (Propan-1,3-diyl), Methylendioxy oder Ethylendioxy,
Cyclopropyl, Cyclobutyl, Cyclopentyl oder Cyclohexyl,
und/oder
durch Phenyl, Phenoxy, Phenylthio, Benzyl, Benzyloxy, Benzylthio, Pyridyl, Pyrimidinyl oder Thienyl, wobei diese Reste einfach bis dreifach, gleichartig oder verschieden substituiert sein können durch
Fluor, Chlor, Brom, Nitro, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Trifluormethyl, Trifluorethyl, Difluormethoxy, Trifluormethoxy, Difluorchlormethoxy und/oder Trifluorethoxy.
- R²: steht besonders bevorzugt für Methyl, Ethyl, n- oder i-Propyl, n-, i-, s-, oder t-Butyl, Dimethylamino, Diethylamino oder Methyl-ethylamino,
- R²: steht auch besonders bevorzugt für gegebenenfalls einfach oder zweifach durch Fluor, Chlor, Brom, Nitro, Methyl, Ethyl und/oder Methoxy substituiertes Phenyl.

Verwendet man beispielsweise 3-Phenoxy-4H-[1,2,4]oxadiazol-5-on und Methylsulfonylchlorid als Ausgangsstoffe, so läßt sich der Verlauf des erfindungsgemäßen Verfahrens durch das folgende Formelschema veranschaulichen:

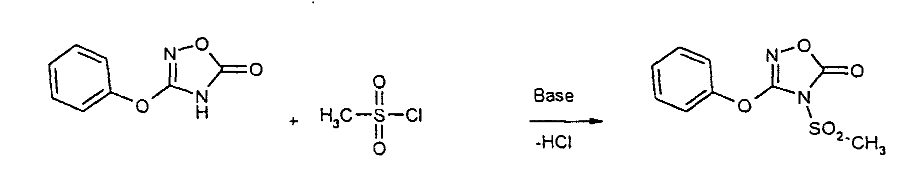

Die zur Durchführung des erfindungsgemäßen Verfahrens als Ausgangsstoffe benötigten Oxadiazolone sind durch die Formel (II) allgemein definiert. In dieser Formel haben A und R¹ vorzugsweise bzw. insbesondere diejenigen Bedeutungen, die bereits im Zusammenhang mit der Beschreibung der erfindungsgemäßen Verbindungen der Formel (I) als bevorzugt bzw. als insbesondere bevorzugt für A und R¹ angegeben wurden.

Die Oxadiazolone der Formel (II) sind bekannt oder können nach bekannten Methoden hergestellt werden (vergleiche Chem. Ber. 98 (1965), 144-154).

Die zur Durchführung des erfindungsgemäßen Verfahrens als Reaktionskomponenten benötigten Sulfonsäurehalogenide sind durch die Formel (III) allgemein definiert. In dieser Formel hat R² vorzugsweise bzw. insbesondere diejenige Bedeutung, die bereits im Zusammenhang mit der Beschreibung der erfindungsgemäßen Verbindungen der Formel (I) als bevorzugt bzw. als insbesondere bevorzugt für R² angegeben wurde. X steht vorzugsweise für Chlor.

Die Sulfonsäurehalogeriide der Formel (III) sind übliche Laborchemikalien.

Als Verdünnungsmittel zur Durchrührung des erfindungsgemäßen Verfahrens kommen alle inerten organischen Lösungsmittel in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofüran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Ester wie Essigsäuremethylester oder Essigsäureethylester.

Als Säurebindemittel kommen bei der Durchführung des erfindungsgemäßen Verfahrens alle üblichen anorganischen oder organischen Basen infrage. Vorzugsweise verwendbar sind Erdalkalimetall- oder Alkalimetallhydride, -hydroxide, -amide, -alkoholate, -acetate, -carbonate oder -hydrogencarbonate, wie Natriumhydrid, Natriumamid, Natrium-methylat, Natrium-ethylat, Kalium-tert-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, ferner Ammonium-Verbindungen, wie Ammoniumhydroxid, Ammoniumacetat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Katalysatoren kommen bei der Durchführung des erfindungsgemäßen Verfahrens alle für derartige Umsetzungen üblichen Reaktionsbeschleuniger in Betracht. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄alkyl-benzylammoaiumchlorid, Dimethyl-C₁₂/C₁₄-alkyl-benzyl-ammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 20°C und 120°C.

Zur Durchführung des erfindungsgemäßen Verfahrens setzt man pro Mol an Oxadiazolon der Formel (II) im allgemeinen 1 bis 2 Mol, vorzugsweise 1 bis 1,3 Mol an Sulfonsäurehalogenid der Formel (III) und gegebenenfalls 1,0 bis 2,0 Mol, vorzugsweise 1,0 bis 1,3 Mol an Säureakzeptor ein.

Das erfindungsgemäße Verfahren wird im allgemeinen unter Atmosphärendruck durchgerührt. Es ist jedoch auch möglich, unter erhöhtem oder vermindertem Druck - im allgemeinen zwischen 0, 1 bar und 10 bar - zu arbeiten.

Die Aufarbeitung erfolgt nach üblichen Methoden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz eingesetzt werden.

Fungizide lassen sich Pflanzenschutz zur Bekämpfung von Plasmodiophoromycetes, Oomycetes, Chytridiomycetes, Zygomycetes, Ascomycetes; Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca füliginea;
Podosphaera-Arten, wie beispielsweise Podosphaera leucotricha;
Verituria-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Phytophthora-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden; Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofüram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazole, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5 -(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester
1-(2,4-Dichlorphenyl)-2-(1H-1,2,4-triazol-1-yl}-ethanon-O-(phenylmethyl)-oxim,
1-(2-Methyl-1-naphthalenyl)-1H-pyrrol-2,5-dion,
1-(3, 5-Dichlorphenyl)-3-(2-propenyl)-2, 5 -pyrrolidindion,
1-[(Diiodmethyl)-sulfonyl]-4-methyl-benzol,
1-[[2-(2,4-Dichlorphenyl)-1,3-dioxolan-2-yl]-methyl]-1 H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl)-ethenyl]- 1H-imidazol,
1-Methyl-5-nonyl-2-(phenylmethyl)-3-pyrrolldinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridiacarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl-2-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1 -Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3 -methyl-4-[(3 -methylbenzoyl)-oxy)-2, 5-thiophendicarboxylat,
cis-1-(4-Chlorphenyl)-2-( 1 H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2, 6-Dimethylphenyl)-2-methoxy-N-(tetrahydro-2-oxo-3-füranyl)-acetarnid,
N-(2,6-Dimethylphenyi)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3 -pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin -Natriumsalz,
O,O-Diethyi-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1(3'H)-isobenzofuran]-3'-on,

### Bakterizide:

Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephät, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1 -(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Pröpaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßeü Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Herstellung und die Verwendung von erfindungsgemäßen Wirkstoffen werden durch die folgenden Beispiele veranschaulicht.

### Herstellungsbeispiele:

### Beispiel 1

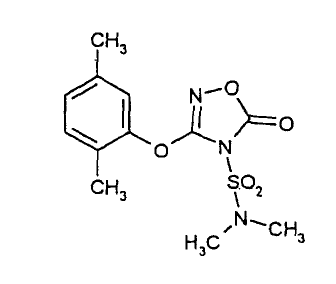

Eine Lösung aus 2,062 g (0,01 Mol) 3-(2,4-Dimethylphenoxy)-4H-[1,2,4]oxadiazol-5-on, 1,44 g (0,01 Mol) Dimethylaminosulfonylchlorid und 1,52 g (0.015 Mol) Triethylamin in 50 ml Chloroform wird 7 Tage bei Raumtemperatur gerührt. Danach wird die Mischung so lange mit Wasser gewaschen, bis das Waschwasser neutral reagiert. Die Lösung wird über Magnesiumsulfat getrocknet und unter vermindertem Druck eingeengt. Das verbleibende Produkt wird aus Ethanol/Hexan (1:2) umkristallisiert. Man erhält 1,4 g (44,73 % der Theorie) an 3-(2,5-Dimethylphenoxy)-5-oxo-[1,2,4]oxadiazol-4-sulfonsäuredimethylamid vom Schmelzpunkt 101 - 103°C.

### Beispiel 2

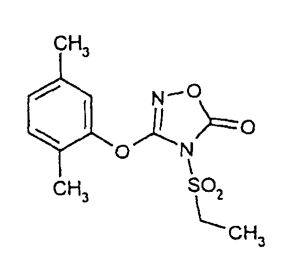

Eine Mischung aus 2,062 g (0,01 Mol) 3-(2,4-Dimethylphenoxy)-4H-[1,2,4]oxadiazol-5-on, 1,29 g (0,01 Mol) Ethansulfonylchlorid, 0,6 g (0.015 Mol) Natriumhydroxid und 0,1 g Tetrabutylammoniumbromid in 50 ml Chloroform wird 22 Stunden bei Raumtemperatur gerührt. Danach wird die Mischung so lange mit Wasser gewaschen, bis das Waschwasser neutral reagiert. Die Lösung wird dann über Magnesiumsulfat getrocknet und unter vermindertem Druck eingeengt. Das anfallende Produkt wird aus Ethanol/Hexan (1:2) umkristallisiert. Man erhält 1,2 g (40,27 % der Theorie) an 3-(2,5-Dimethylphenoxy)-4-ethansulfonyl-4H-[1,2,4]oxadiazol-5-on vom Schmelzpunkt 67-69°C.

Nach den zuvor angegebenen Methoden werden auch die in der folgenden Tabelle aufgeführten Sulfonyloxadiazolone der Formel (I) hergestellt.

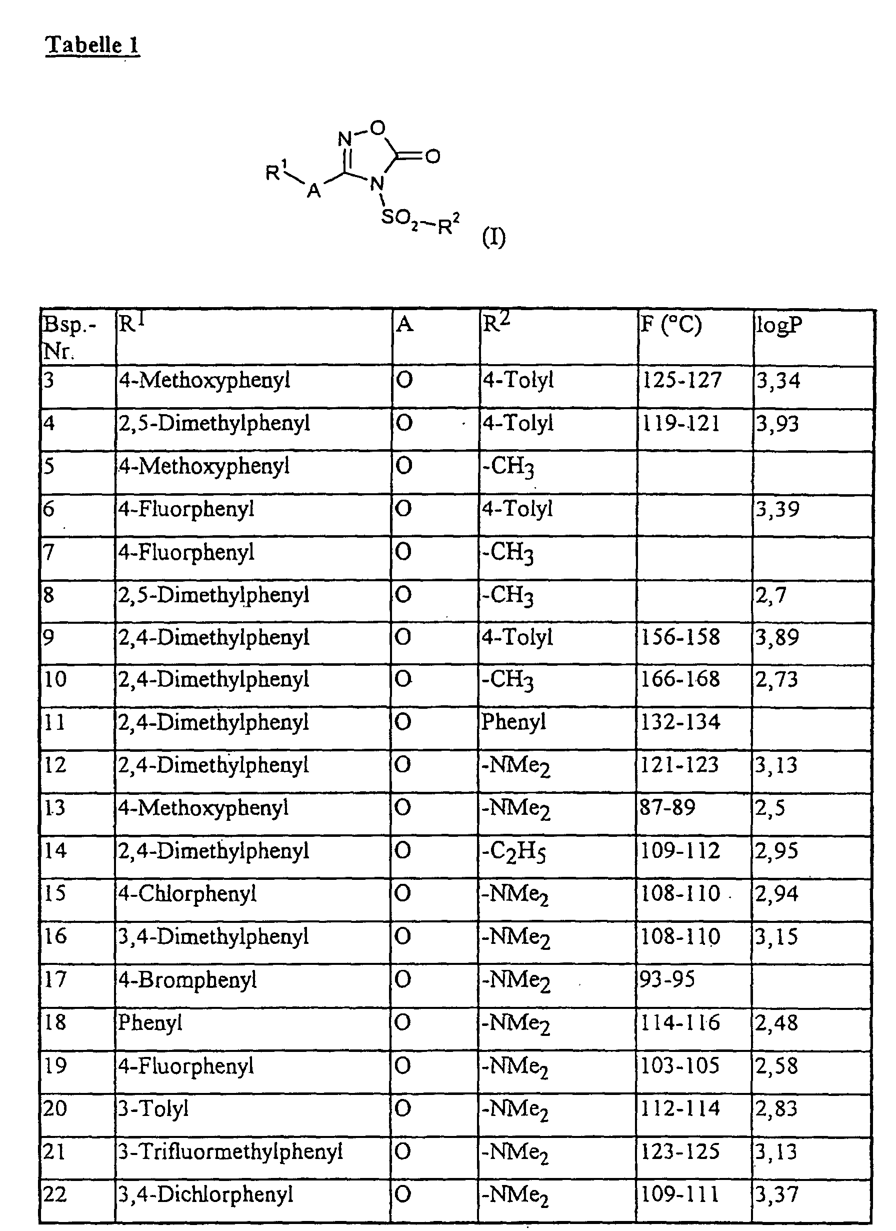

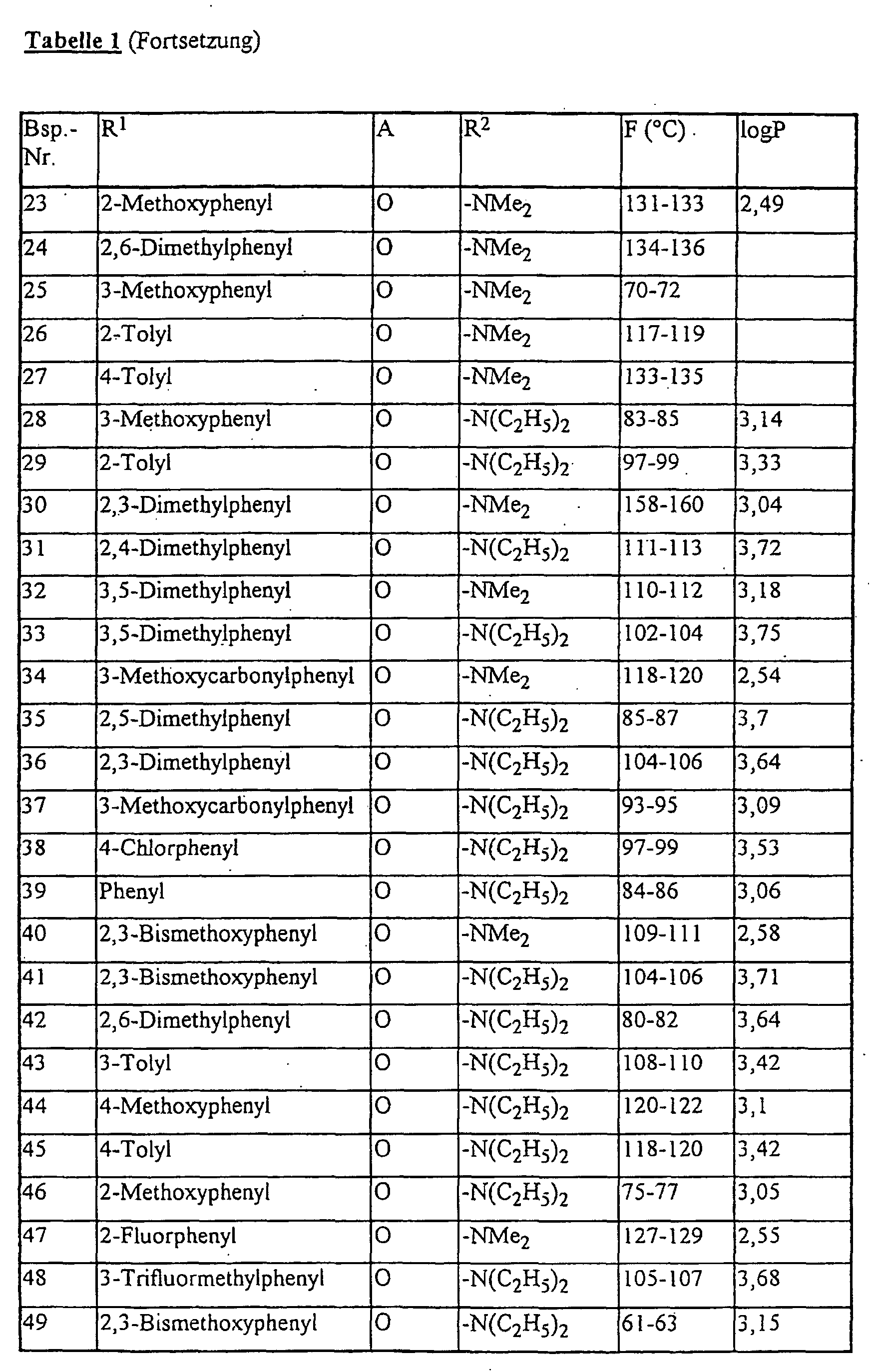

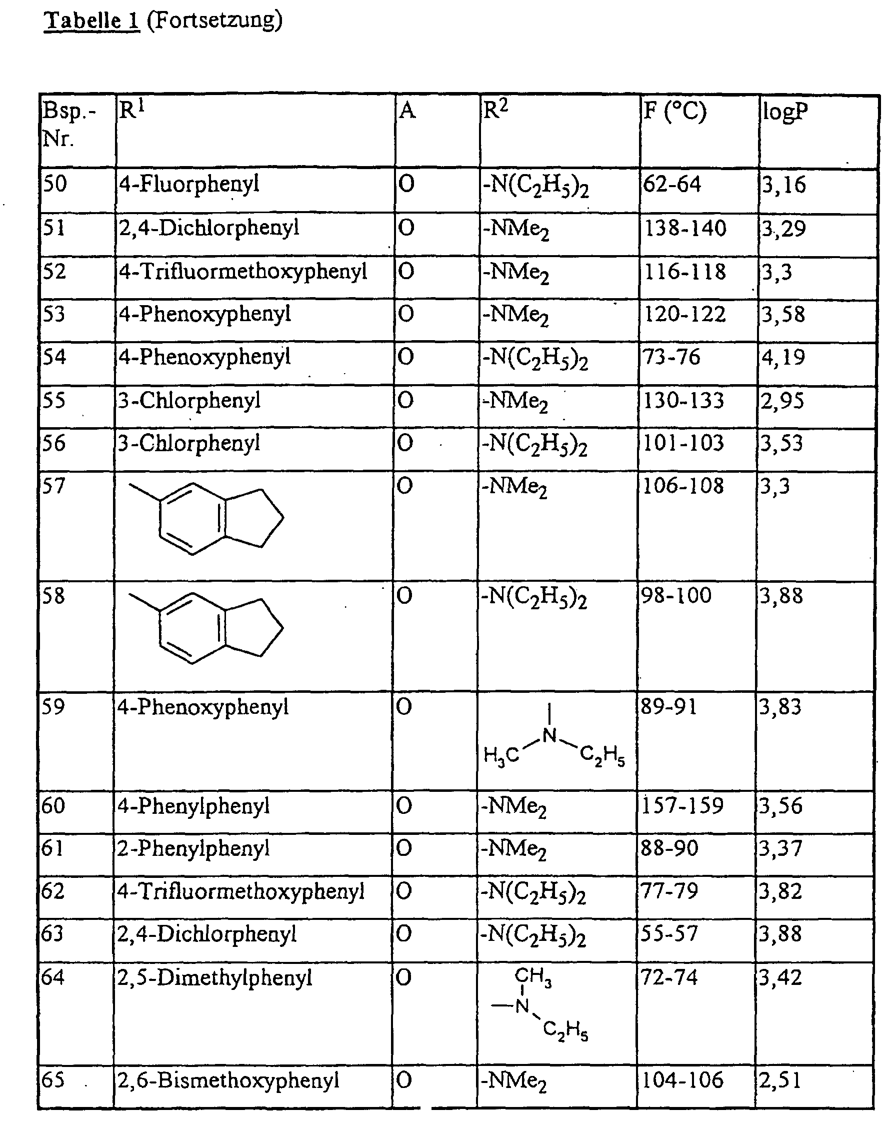

In der Tabelle 1 steht Me für Methyl. Die Bestimmung der logP-Werte erfolgte gemäß EEC-Directive 79/831 Annex V. A8 durch HPLC (Gradientenmethode, Acetonitril/0,1 % wäßrige Phosphorsäure).

### Verwendungsbeispiele

### Beispiel A

| Phytophthora-Test (Tomate) / protektiv | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Phytophthora infestans inokuliert. Die Pflanzen werden dann in einer Inkubationskabine bei ca. 20°C und 100 % relativer Luftfeuchtigkeit aufgestellt

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

Wirkstoffe, Aufwandmengen und Versuchsergebnisse gehen aus der folgenden Tabelle hervor.

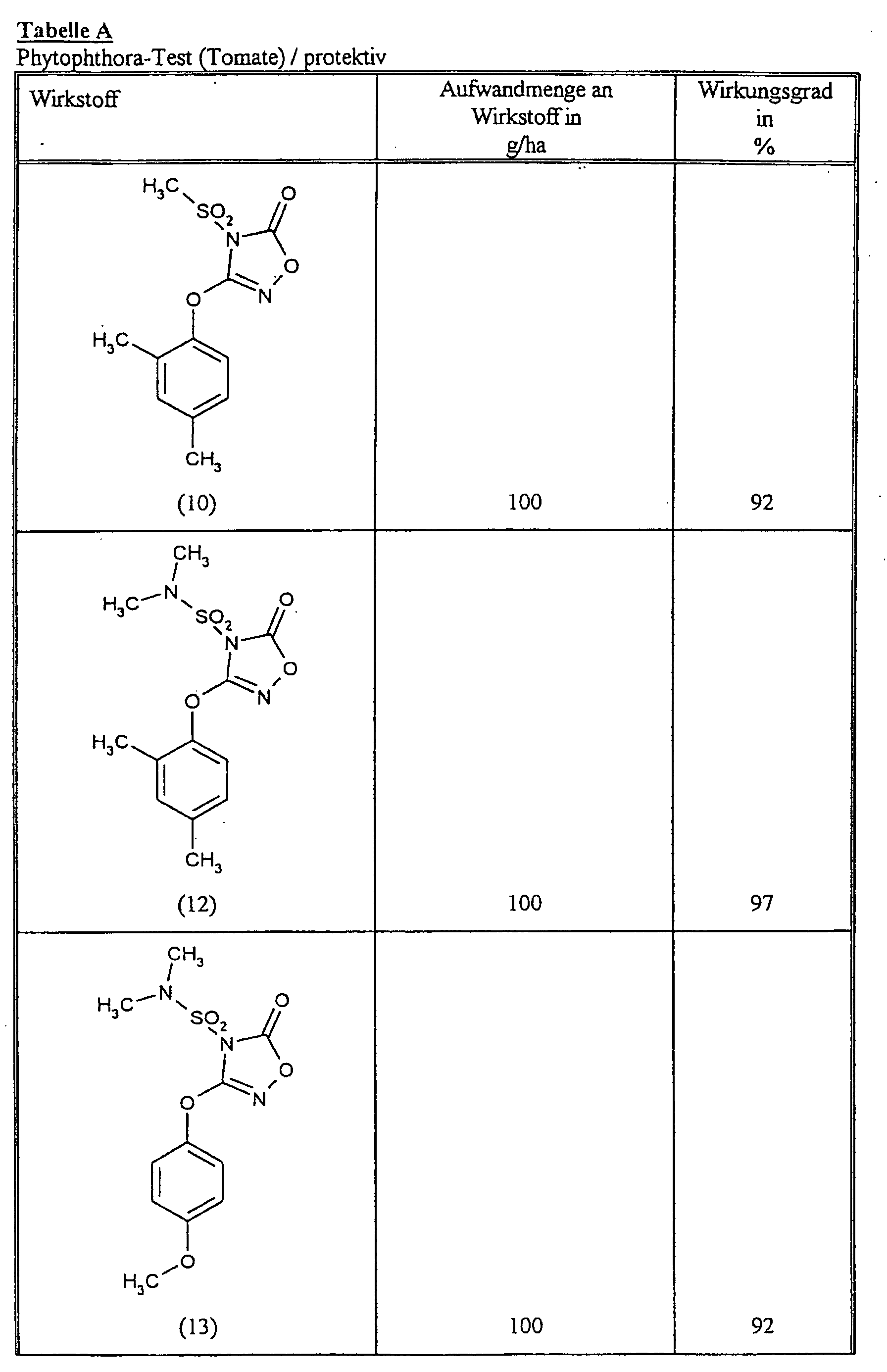

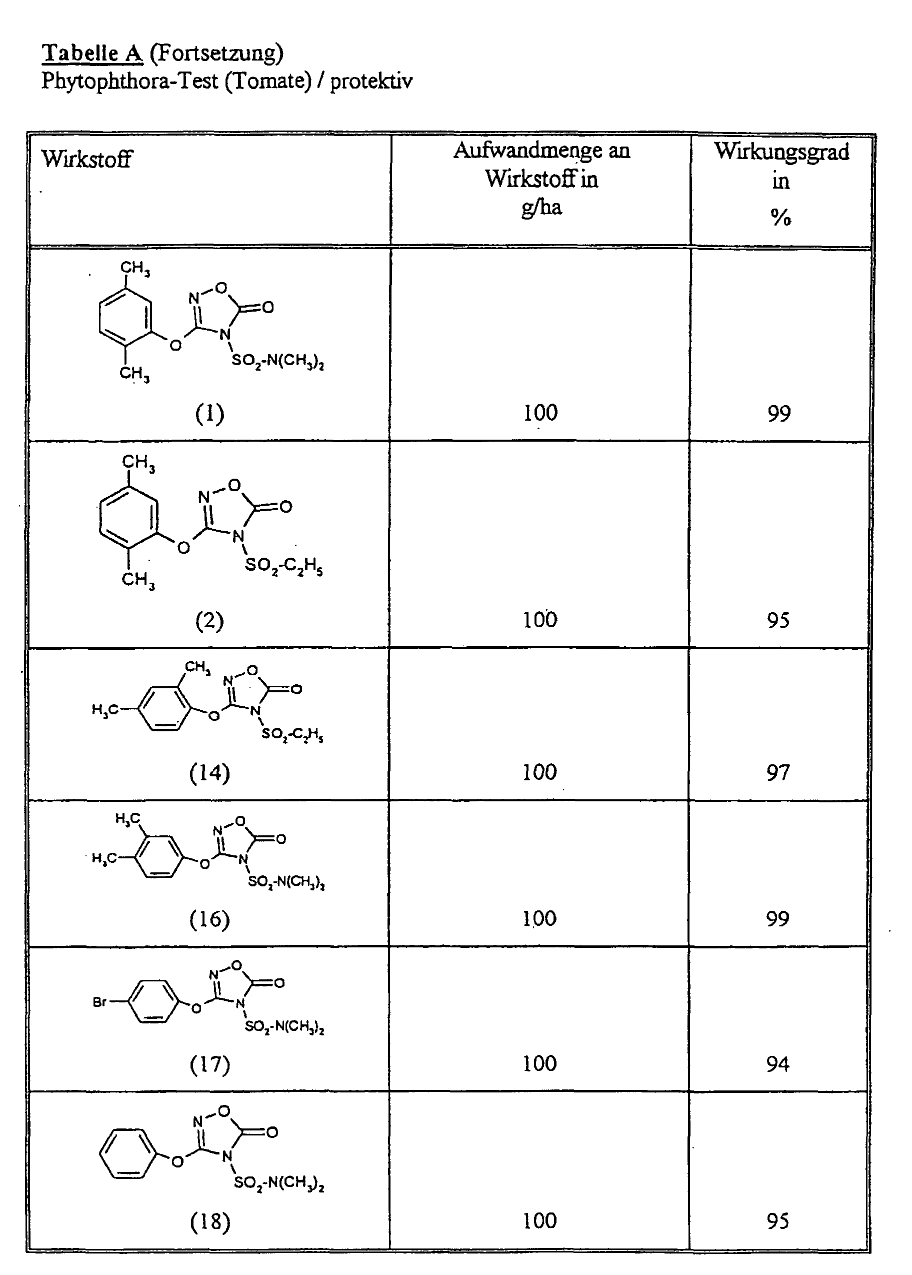

## Patentansprüche

1. Sulfonyloxadiazolone der Formel 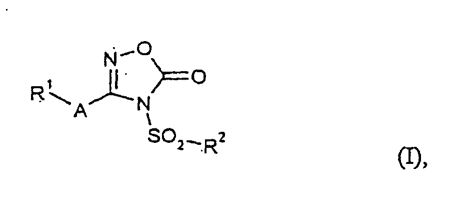 in welcher
A für Sauerstoff Schwefel, -SO-, -SO₂- oder 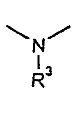 steht, wobei
R³ für Wasserstoff oder Alkyl mit 1 bis 4 Kohlenstoffatomen steht,
R¹ für jeweils gegebenenfalls einfach bis dreifach, gleichartig oder verschieden durch Halogen oder Alkyl mit 1 bis 3 Kohlenstoffatomen
substituiertes Cycloalkyl mit 3 bis 8 Kohlenstoffatomen oder Cycloalkenyl mit 3 bis 8 Kohlenstoffatomen steht oder
R¹ für Aryl mit 6 bis 10 Kohlenstoffatomen steht, wobei jeder dieser Reste einfach bis dreifach, gleichartig oder verschieden substituiert sein kann durch
Halogen, Nitro, Carbamoyl;
jeweils geradkettiges oder verzweigtes Alkyl, Alkoxy, Alkylthio oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio oder Halogenalkylsulfonyl mit jeweils 1 bis 6 Kohlenstoffatomen und 1 bis 5 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 5 gleichen oder verschiedenen Halogenatomen;
jeweils geradkettiges oder verzweigtes Alkylamino, Dialkylanojmo, Alkylcarbonyl, Alkoxycarbonyl, Hydröximinoalkyl oder Alkoximinoalkyl mit jeweils 1 bis 6 Kohlenstoffatomen in den einzelnen Alkylteilen;
jeweils gegebenenfalls einfach bis vierfach,- gleich oder verschieden durch Halogen und/oder geradkettiges oder verzweigtes Alkyl mit 1 bis 4 Köhlenstoffatomen und/oder geradkettiges oder verzweigtes Halogenalkyl mit 1 bis 4 Kohlenstoffätomen und 1 bis 5 gleichen oder verschiedenen Halogsnatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
und/oder
Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio, wobei diese aromatischen oder heterocyciischen Reste einfach bis dreifach, gleichartig oder verschieden substituiert sein können durch
Halogen, Nitro, geradkettiges oder. verzweigtes Alkyl mit 1 bis 4 Kohlenstoffatomen,
geradkettiges oder verzweigtes Halogenalkyl mit 1 bis 4 Kohlenstoffatomen und 1 bis 5 gleichen oder verschiedenen Halogenatomen,
geradkettiges oder verzweigtes Alkoxy oder Alkylthio mit 1 bis 4 Kohlenstoffatomen,
und/oder durch geradkettiges oder verzweigtes Halogenalkoxy mit 1 bis 4 Kohlenstoffatomen und 1 bis 9 gleichen oder verschiedenen Halogenatomen,
und
R² für Alkyl mit 1 bis 4 Kohlenstoffatomen, Alkenyl mit 2 bis 4 Kohlenstoffatomen, Halogenalkyl mit jeweils 1 bis 4 Kohlenstoffatomen und 1 bis 5 gleichen oder verschiedenen Halogenatomen, Halogenalkenyl mit jeweils 2 bis 4 Kohlenstoffatomen und 1 bis 5 gleichen oder verschiedenen Halogenatomen oder für Dialkylamino mit jeweils 1 bis 4 Kohlenstoffatomen in den beiden Alkylgruppen steht, oder
R² für Aryl mit 6 bis 10 Kohlenstoffatomen steht, wobei diese Reste einfach bis dreifach, gleichartig oder verschieden substituiert sein können durch Nitro, Halogen, Alkyl mit 1 bis 4 Kohlenstoffatomen, Alkoxy mit 1 bis 4 Kohlenstoffatomen, Alkylthio mit 1 bis 4 Kohlenstofiatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen und/oder Halogenalkylthio mit 1 bis 4 Kohlenstoffatomen und 1 bis 5 gleichen oder verschiedenen Halogenatomen.

2. Sulfonyloxadiazolon gemäß Anspruch 1, **gekennzeichnet durch** die Formel

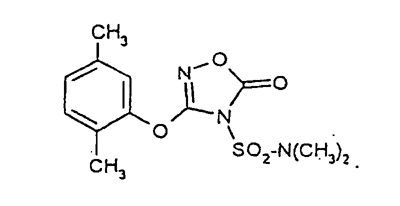

3. Sulfonyloxadiazoton gemäß Anspruch 1, **gekennzeichnet durch** die Formel

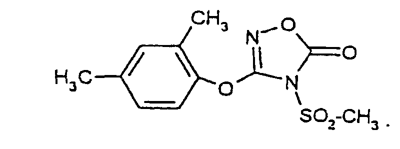

4. Sulfönyloxadiazolon gemäß Anspruch 1, **gekennzeichnet durch** die Formel

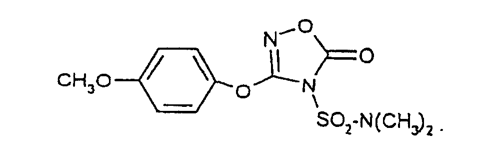

5. Verfahren zur Herstellung von Sulfonyloxadiazolonen der Formel (I) gemäß Anspruch 1, **dadurch gekennzeichnet, daß** man Oxadiazolone der Formel 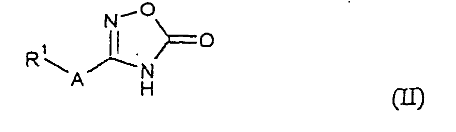 in welcher
A und R¹ die oben angegebenen Bedeutungen haben,
mit Sulfonsäurehalogeniden der Formel
R²-SO₂-X (III)
in welcher
R² die oben angegebene Bedeutung hat und
X für Halogen steht,
gegebenenfalls in Gegenwart eines Säurebindemittels und gegebenenfalls in Gegenwart eines Verdünnungsmittels sowie gegebenenfalls in Gegenwart eines Katalysators umsetzt.

6. Fungizide Mittel, **gekennzeichnet durch** einen Gehalt an mindestens einem Sulfonyloxadiazolon der Formel (I) gemäß Anspruch 1 neben Streckmitteln und/oder oberflächenaktiven Stoffen.

7. Verwendung von Sulfonyloxadiazolonen der Formel (I) gemäß Anspruch 1 al Fungizide im Pflanzenschutz.

8. Verfahren zur Bekämpfung von unerwünschten Pilzen im Pflanzenschutz **dadurch gekennzeichnet, daß** man Sulfonyloxadiazolone der Formel (I) gemäß Anspruch 1 auf die Pilze und/oder deren Lebensraum ausbringt.

9. Verfahren zur Herstellung von fungiziden Mitteln, **dadurch gekennzeichnet, daß** man Sulfonyloxadiazolone der Formel (I) gemäß Anspruch 1 mit Streckmitteln und/oder oberflächenaktiven Stoffen vermischt.

## Claims

1. Sulphonyloxadiazolones of the formula 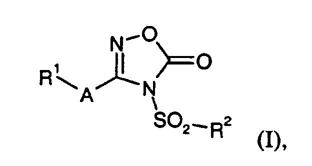 in which
A represents oxygen, sulphur, -SO-, -SO₂- or 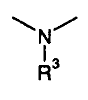 where
R³ represents hydrogen or alkyl having 1 to 4 carbon atoms,
R¹ represents cycloalkenyl having 3 to 8 carbon atoms or cycloalkyl having 3 to 8 carbon atoms which in each case is optionally mono- to trisubstituted by identical or different substituents from the group consisting of halogen and alkyl having 1 to 3 carbon atoms or
R¹ represents aryl having 6 to 10 carbon atoms, where each of these radicals may be mono- to trisubstituted by identical or different substituents from the group consisting of
halogen, nitro, carbamoyl;
in each case straight-chain or branched alkyl, alkoxy, alkylthio or alkylsulphonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio or halogenoalkylsulphonyl having in each case 1 to 6 carbon atoms and 1 to 5 identical or different halogen atoms;
in each case straight-chain or branched halogenoalkenyl or halogenoalkenyloxy having in each case 2 to 6 carbon atoms and 1 to 5 identical or different halogen atoms;
in each case straight-chain or branched alkylamino, dialkylamino, alkylcarbonyl, alkoxycarbonyl, hydroximinoalkyl or alkoximinoalkyl having in each case 1 to 6 carbon atoms in the individual alkyl moiety;
in each case doubly attached alkylene or dioxyalkylene having in each case 1 to 6 carbon atoms and being in this case optionally mono- to tetrasubstituted by identical or different substituents from the group consisting of halogen and straight-chain or branched alkyl having 1 to 4 carbon atoms and straight-chain or branched halogenoalkyl having 1 to 4 carbon atoms and 1 to 5 identical or different halogen atoms;
cycloalkyl having 3 to 6 carbon atoms;
and
aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, where these aromatic or heterocyclic radicals may be mono- to trisubstituted by identical or different substituents from the group consisting of
halogen, nitro, straight-chain or branched alkyl having 1 to 4 carbon atoms,
straight-chain or branched halogenoalkyl having 1 to 4 carbon atoms and 1 to 5 identical or different halogen atoms,
straight-chain or branched alkoxy or alkylthio having 1 to 4 carbon atoms,
and by straight-chain or branched halogenoalkoxy having 1 to 4 carbon atoms and 1 to 9 identical or different halogen atoms,
and
R² represents alkyl having 1 to 4 carbon atoms, alkenyl having 2 to 4 carbon atoms, halogenoalkyl having in each case 1 to 4 carbon atoms and 1 to 5 identical or different halogen atoms, halogenoalkenyl having in each case 2 to 4 carbon atoms and 1 to 5 identical or different halogen atoms or represents dialkylamino having in each case 1 to 4 carbon atoms in the two alkyl groups, or
R² represents aryl having 6 to 10 carbon atoms, where these radicals may be mono- to trisubstituted by identical or different substituents from the group consisting of nitro, halogen, alkyl having 1 to 4 carbon atoms, alkoxy having 1 to 4 carbon atoms, alkylthio having 1 to 4 carbon atoms, halogenoalkyl having 1 to 4 carbon atoms and 1 to 5 identical or different halogen atoms, halogenoalkoxy having 1 to 4 carbon atoms and 1 to 5 identical or different halogen atoms and halogenoalkylthio having 1 to 4 carbon atoms and 1 to 5 identical or different halogen atoms.

2. Sulphonyloxadiazolone according to Claim 1, **characterized by** the formula

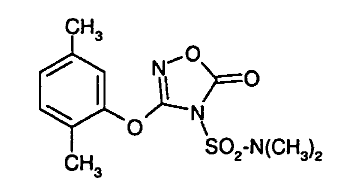

3. Sulphonyloxadiazolone according to Claim 1, **characterized by** the formula

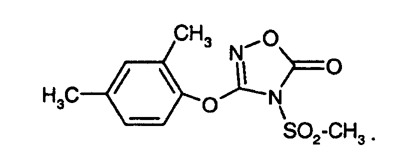

4. Sulphonyloxadiazolone according to Claim 1, **characterized by** the formula

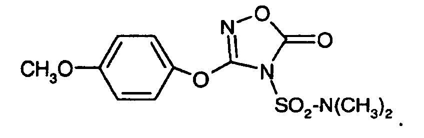

5. Process for preparing sulphonyloxadiazolones of the formula (I) according to Claim 1, **characterized in that** oxadiazolones of the formula 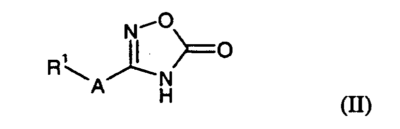 in which
A and R¹ are as defined above,
are reacted with sulphonyl halides of the formula
R²-SO₂-X (III)
in which
R² is as defined above and
X represents halogen,
if appropriate in the presence of an acid binder and if appropriate in the presence of a diluent and if appropriate in the presence of a catalyst.

6. Fungicidal compositions, **characterized in that** they contain at least one sulphonyloxadiazolone of the formula (I) according to Claim 1, in addition to extenders and/or surfactants.

7. Use of sulphonyloxadiazolones of the formula (I) according to Claim 1 as fungicides in crop protection.

8. Method for controlling undesirable fungi in crop protection, **characterized in that** sulphonyloxadiazolones of the formula (I) according to Claim 1 are applied to the fungi and/or their habitat.

9. Process for preparing fungicidal compositions, **characterized in that** sulphonyloxadiazolones of formula (I) according to Claim 1 are mixed with extenders and/or surfactants.

## Revendications

1. Sulfonyloxadiazolones de formule 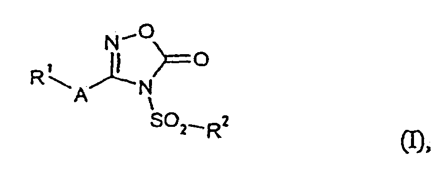 dans laquelle
A représente l'oxygène, le soufre, un groupe -SO-, -SO₂- ou un groupe 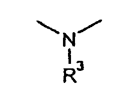
dans lequel
R³ représente l'hydrogène ou un reste alkyle ayant 1 à 4 atomes de carbone,
R¹ est un reste cycloalkyle ayant 3 à 8 atomes de carbone ou un reste cycloalcényle ayant 3 à 8 atomes de carbone, chacun portant éventuellement 1 à 3 substituants, identiques ou différents, halogéno ou alkyle ayant 1 à 3 atomes de carbone, ou bien
R¹ est un reste aryle ayant 6 à 10 atomes de carbone, pouvant porter dans chaque cas 1 à 3 substituants, identiques ou différents,
halogéno, nitro, carbamoyle;
un reste alkyle, alkoxy, alkylthio ou alkylsulfonyle ayant chacun 1 à 6 atomes de carbone et chacun étant linéaire ou ramifié ;
un reste alcényle ou alcényloxy ayant chacun 2 à 6 atomes de carbone et chacun étant linéaire ou ramifié ;
un reste halogénalkyle, halogénalkoxy, halogénalkylthio ou halogénalkylsulfonyle ayant chacun 1 à 6 atomes de carbone et 1 à 5 atomes d'halogène identiques ou différents et chacun étant linéaire ou ramifié ;
un reste halogénalkyle ou halogénalkoxy ayant chacun 2 à 6 atomes de carbone et 1 à 5 atomes d'halogène identiques ou différents et chacun étant linéaire ou ramifié ;
un reste alkylamino, dialkylamino, alkylcarbonyle, alkoxycarbonyle, hydroximino-alkyle ou alkoximinoalkyle ayant chacun 1 à 6 atomes de carbone dans les parties alkyle individuelles et chacun étant linéaire ou ramifié ;
un reste alkylène ou dioxyalkylène ayant chacun 1 à 6 atomes de carbone, chacun ayant deux liaisons et chacun portant, le cas échéant, 1 à 4 substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 5 atomes d'halogène identiques ou différents ;
un reste cycloalkyle ayant 3 à 6 atomes de carbone ;
et/ou
un reste aryle, aryloxy, arylthio, arylalkyle, arylalkyloxy, arylalkylthio, hétérocyclyle, hétérocyclyloxy, hétérocyclylthio, hétérocyclylalkyle, hétéro- cyclylalkyloxy ou hétérocyclylalkylthio, chacun de ces restes aromatiques ou hétérocycliques pouvant porter 1 à 3 substituants, identiques ou différents :
halogéno, nitro, alkyle linéaire ou ramifié ayant 1 à 4 atomes de carbone,
halogénalkyle linéaire ou ramifié ayant 1 à 4 atomes de carbone et 1 à 5 atomes d'halogène identiques ou différents,
alkoxy ou alkylthio linéaire ou ramifié ayant 1 à 4 atomes de carbone,
et/ou halogénalkoxy linéaire ou ramifié ayant 1 à 4 atomes de carbone et 1 à 9 atomes d'halogène identiques ou différents,
et
R² représente un reste alkyle ayant 1 à 4 atomes de carbone, alcényle ayant 2 à 4 atomes de carbone, halogénalkyle ayant chacun 1 à 4 atomes de carbone et 1 à 5 atomes d'halogène identiques ou différents, halogénalcényle ayant 2 à 4 atomes de carbone et 1 à 5 atomes d'halogène identiques ou différents ou dialkylamino ayant chacun 1 à 4 atomes de carbone dans chacun des deux groupes alkyle, ou bien
R² est un reste aryle ayant 6 à 10 atomes de carbone, ces restes aryle pouvant porter 1 à 3 substituants, identiques ou différents, nitro, halogéno, alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogène identiques ou différents, halogénalkoxy ayant 1 à 4 atomes de carbone et 1 à 5 atomes d'halogène identiques ou différents et/ou halogénalkylthio ayant 1 à 4 atomes de carbone et 1 à 5 atomes d'halogène identiques ou différents.

2. Sulfonyloxadiazolone suivant la revendication 1, **caractérisée par** la formule

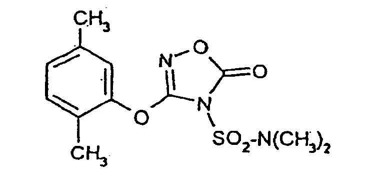

3. Sulfonyloxadiazolone suivant la revendication 1, **caractérisée par** la formule

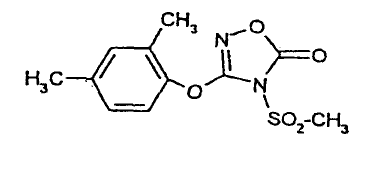

4. Sulfonyloxadiazolone suivant la revendication 1, **caractérisée par** la formule

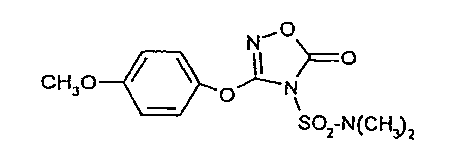

5. Procédé de production de sulfonyloxadiazolones de formule (I) suivant la revendication 1, **caractérisé en ce qu'**on fait réagir des oxadiazolones de formule 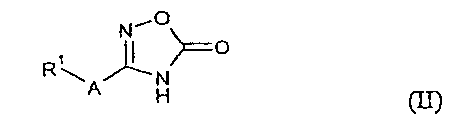 dans laquelle
A et R¹ ont les définitions indiquées ci-dessus,
avec des halogénures d'acides sulfoniques de formule
R²-SO₂-X (III)
dans laquelle
R² a la définition indiquée ci-dessus et
X est un halogène,
en présence éventuelle d'un accepteur d'acide et, le cas échéant, en présence d'un diluant ainsi que, le cas échéant, en présence d'un catalyseur.

6. Compositions fongicides, **caractérisées par** une teneur en au moins une sulfonyloxadiazolone de formule (I) suivant la revendication 1, à côté de diluants et/ou de substances tensio-actives.

7. Utilisation de sulfonyloxadiazolones de formule (I) suivant la revendication 1, comme fongicides dans la protection de plantes.

8. Procédé pour combattre des champignons indésirables dans la protection de plantes, **caractérisé en ce qu'**on épand des sulfonyloxadiazolones de formule (I) suivant la revendication 1 sur les champignons et/ou sur leur milieu.

9. Procédé de préparation de compositions fongicides, **caractérisé en ce qu'**on mélange des sulfonyloxadiazolones de formule (I) suivant la revendication 1 avec des diluants et/ou des substances tensio-actives.
